# EUROPÄISCHE PATENTANMELDUNG

(11) **EP 3 355 100 A1**
(43) Veröffentlichungstag der Anmeldung: **01.08.2018**
(21) Anmeldenummer: 17153556.0
(22) Anmeldetag: 27.01.2017
(51) Int. Cl.: G02C 13/00

(54) **VORRICHTUNG ZUR BESTIMMUNG VON ZENTRIERPARAMETERN FÜR DIE BRILLENANPASSUNG**

(71) Anmelder: Carl Zeiss Vision International GmbH, 73430 Aalen (DE); Carl Zeiss AG, 73447 Oberkochen (DE)
(72) Erfinder: ALVAREZ DIEZ, Cristina, 73447 Oberkochen (DE); BREUNINGER, Tobias, 89542 Herbrechtingen (DE); GAMPERLING, Michael, 89340 Leipheim (DE); SCHWARZ, Oliver, 73492 Rainau (DE); WIDULLE, Frank, 89233 Neu-Ulm (DE)
(74) Vertreter: Patentanwälte Bregenzer und Reule Partnerschaftsgesellschaft mbB

(57) **Zusammenfassung**

Die Erfindung betrifft eine Vorrichtung (10) zur Bestimmung von Zentrierparametern für die Brillenanpassung mit einem Kameraträger (14), welcher einen nach oben, nach unten und zu einer Rückseite (30) hin offenen Innenraum (22) teilweise umschließt und mindestens drei Kameras (16a, 16b) trägt, die zwischen zwei freien Enden (18) des Kameraträgers (14) angeordnet und auf den Innenraum (22) gerichtet sind, wobei der Kameraträger (14) zur Beleuchtung des Innenraums (22) eine Beleuchtungseinrichtung (32, 34, 36) aufweist.

## Beschreibung

Die Erfindung betrifft eine Vorrichtung zur Bestimmung von Zentrierparametern für die Brillenanpassung.

Zentrierparameter werden benutzt, um Brillengläser korrekt in einer Brillenfassung anzuordnen bzw. zu zentrieren, so dass die Brillengläser in korrekter Position relativ zu den Augen der die Brille tragenden Person angeordnet sind. Dabei handelt es sich zum Teil um anatomische Parameter der betreffenden Person, wie beispielsweise den Pupillenabstand, zum Teil um rein fassungsspezifische Parameter wie die Fassungsscheibenbreite oder die Fassungsscheibenhöhe und zum Teil um Kombinationen aus anatomischen und fassungsspezifischen Parametern, wie beispielsweise den Hornhautscheitelabstand und die Durchblickshöhe. Einen Überblick über die gängigen Zentrierparameter gibt die DIN EN ISO 13666 vom Oktober 2013.

Es sind Vorrichtungen bekannt, mit denen eine automatische Bestimmung von Zentrierparametern möglich ist. So ist beispielsweise aus der EP 1 844 363 B2 eine solche Vorrichtung bekannt, bei der mittels zweier Kameras aus unterschiedlichen Blickrichtungen Bilder einer eine Brille bzw. Brillenfassung tragenden Person aufgenommen werden, so dass aus den aufgenommenen Bilddaten Zentrierparameter bestimmt werden können.

Bei bekannten Vorrichtungen zur Bestimmung von Zentrierparametern besteht jedoch oft das Problem, dass die aus unterschiedlichen Blickrichtungen aufgenommenen Bilder in Ihrer Qualität und Verwertbarkeit schwanken.

Es ist daher Aufgabe der Erfindung, eine Vorrichtung der eingangs genannten Art derart weiterzubilden, dass bessere Bilder der Person, für die die Zentrierparameter bestimmt werden sollen, erzeugt werden können.

Diese Aufgabe wird erfindungsgemäß durch eine Vorrichtung mit den Merkmalen des Anspruches 1 gelöst. Vorteilhafte Weiterbildungen der Erfindung sind Gegenstand der abhängigen Ansprüche.

Erfindungsgemäß weist die Vorrichtung einen Kameraträger auf, der einen Innenraum teilweise umschließt, diesen aber nach oben, nach unten und zu einer Rückseite hin offen lässt. Ein Proband kann sich dann von der Rückseite her in den Innenraum bewegen. Der Innenraum wird durch den Kameraträger nach vorne und zu den Seiten begrenzt. Die Kameras sind auf den Innenraum gerichtet. Sie sind in einer Kameraanordnung angeordnet, die sich zwischen zwei freien Enden des Kameraträgers erstreckt. Vorzugsweise erstreckt sich eine konkave Biegung des Kameraträgers in derselben Richtung, in der sich die Kameraanordnung zwischen den freien Enden erstreckt. Dabei ist es möglich, eine kontinuierliche und konkave Biegung vorzusehen, dem Kameraträger beispielsweise in Draufsicht eine Kreisbogenform zu geben, oder aber einen eckigen Grundriss vorzusehen, so dass der Kameraträger eine Frontpartie und in einem Winkel von der Frontpartie abstehende Seitenpartien aufweist. Die Kameras der Kameraanordnung können sämtlich auf gleicher Höhe in einer Kamerareihe angeordnet oder in ihrer Höhe gegeneinander versetzt sein. Um den Innenraum gut auszuleuchten, weist der Kameraträger eine Beleuchtungseinrichtung auf, die sich vorzugsweise zwischen den freien Enden über eine Länge erstreckt, die mindestens der in einer Umfangsrichtung zwischen den freien Enden gemessenen Länge der Kameraanordnung entspricht. Zweckmäßig weist die Beleuchtungseinrichtung mindestens eine Lichtleiste oder Leuchtmittelreihe mit einer Vielzahl von Leuchtmitteln auf. Die Leuchtmittel sind in einer oder mehreren Reihen angeordnet. Die mindestens eine Lichtleiste mit ihrer Vielzahl von Leuchtmitteln, die vorzugsweise als LEDs ausgebildet sind, ermöglicht eine in hohem Maße gleichmäßige Ausleuchtung des Innenraums, auf den die Kameras gerichtet sind. Insbesondere wird angestrebt, dass die Beleuchtungseinrichtung so ausgestaltet ist, dass die auf einer vorbestimmten Oberfläche im Innenraum, insbesondere an einer mittig im Innenraum angeordneten Zylindermantelfläche mit 20 cm Durchmesser, gemessene Lichtintensität an jeder Stelle der Oberfläche um maximal +50% und -30% von einem vorgegebenen Basiswert abweicht. Die Homogenität des Lichts im Innenraum wird weiter verbessert, wenn die Leuchtmittel ihr Licht durch mindestens ein großflächiges Fenster in den Innenraum emittieren. Reflexionen in den Pupillen sind dann, wenn sie überhaupt auftreten, sehr schwach und stören die Zentriermessung kaum. Alle Leuchtmittel weisen zudem vorzugsweise eine im wesentlichen einheitliche Farbtemperatur von ca. 4000 K +/- 1000 Kauf.

Um die Ausleuchtung des Innenraums weiter zu verbessern, wird bevorzugt, dass die Beleuchtungseinrichtung eine erste, über der Kameraanordnung angeordnete, und eine zweite, unter der Kameraanordnung angeordnete Lichtleiste aufweist. Weiter wird bevorzugt, dass die erste und die zweite Lichtleiste jeweils nahe den freien Enden des Kameraträgers durch vertikal verlaufende weitere Lichtleisten miteinander verbunden sind. Die Kameraanordnung wird dann an der Innenfläche des Kameraträgers ringsum durch die Leuchtmittel der Lichtleisten umrahmt, welche sich bevorzugt über einen Mittelpunktswinkel von mindestens 180 Grad erstrecken. Der Abstand der oberen Lichtleiste von der unteren Lichtleiste wird vorzugsweise so gewählt, dass die oben erwähnte Homogenität der Ausleuchtung mit der Abweichung von maximal +50% und -30% vom Basiswert in einem Bereich gegeben ist, der eine Höhe von ca. 20cm hat.

Um aussagefähige Bilddaten erzeugen zu können, wird bevorzugt, dass sich die Anordnung der Kameras über einen Mittelpunktswinkel von mindestens 150 Grad erstreckt bzw. dass die optischen Achsen der dem freien Ende des Kameraträgers am nächsten gelegenen Kameras einen Winkel von mindestens 150 Grad einschließen. Es ist dann möglich, Bilder des Probanden nicht nur im Wesentlichen von vorn, sondern auch von beiden Seiten aufzunehmen. Zudem wird bevorzugt, dass sich die freien Enden des Kameraträgers in einem Abstand von mindestens 20cm und vorzugsweise von mindestens 25cm zueinander befinden. Ein Proband mit durchschnittlich breitem Kopf kann sich dann bequem von der Rückseite in den Innenraum bewegen.

Zweckmäßig weist die Kameraanordnung eine ungerade Zahl von Kameras auf mit einer mittig angeordneten Frontkamera und bezüglich einer durch die optische Achse der Frontkamera verlaufenden Symmetrieebene symmetrisch angeordneten Seitenkameras. Je mehr Kameras die Kameraanordnung enthält, desto genauer können die Zentrierparameter bestimmt werden.

Vorteilhaft ist eine Steuer- oder Regeleinrichtung zum automatischen oder manuellen Steuern oder Regeln der Lichtintensität der Leuchtmittel in Abhängigkeit von der Helligkeit im Innenraum und vorzugsweise in Abhängigkeit von der mittels der Kameras detektierten Helligkeit vorgesehen. Dabei wird bevorzugt, dass Sektoren der Beleuchtungseinrichtung und/oder einzelne Leuchtmittel getrennt voneinander steuerbar oder regelbar sind. Dadurch wird der Tatsache Rechnung getragen, dass in der Praxis regelmäßig Fremdlicht auf den Probanden einfällt, beispielsweise durch ein Fenster des Raumes, in dem sich die Vorrichtung befindet. Um eine gleichmäßige Ausleuchtung des Probanden zu gewährleisten, kann es dann erforderlich sein, beispielsweise die dem Fenster zugewandte Gesichtshälfte weniger stark, die dem Fenster abgewandte Gesichtshälfte aber umso stärker auszuleuchten.

Eine weitere vorteilhafte Weiterbildung sieht einen Abstandssensor vor, der der Messung des Abstands des Kopfes des Probanden zur Mitte des Kameraträgers dient. Zudem ist eine Anzeigeeinheit zur Anzeige des Abstands vorgesehen. Dadurch wird dem Probanden eine Positionierung am gewünschten Ort, beispielsweise in der Mitte des Innenraums, erleichtert. Während der Proband eine Positionierung in der optischen Achse der in der Mitte des Kameraträgers angeordneten Frontkamera meist selbst hinreichend genau vornehmen kann, kann er den Abstand zur Mitte des Kameraträgers meist nur schlecht abschätzen. Der Abstandssensor ermittelt diesen Abstand und zeigt dem Probanden an, ob er zu weit vorne, zu weit hinten oder richtig steht. Dabei kann ein zusätzlicher Abstandssensor vorgesehen sein, oder einige der Kameras bilden derart den Abstandssensor, dass sie aus unterschiedlichen Winkeln den Probanden aufnehmen und durch ein geeignetes Abstandsmessverfahren den Abstand bestimmen.

Vorteilhaft ist am Kameraträger eine Fixationseinrichtung zum Erzeugen eines Fixationsmusters für den Probanden angeordnet, vorzugsweise eine zumindest ein Specklemuster als Fixationsmuster erzeugende Fixationseinrichtung. Eine solche Fixationseinrichtung ist ausführlich in der EP 1 704 437 B1 beschrieben, auf die in diesem Zusammenhang ausdrücklich Bezug genommen wird.

Im Folgenden wird die Erfindung anhand eines in der Zeichnung schematisch dargestellten Ausführungsbeispiels näher erläutert. Es zeigt:

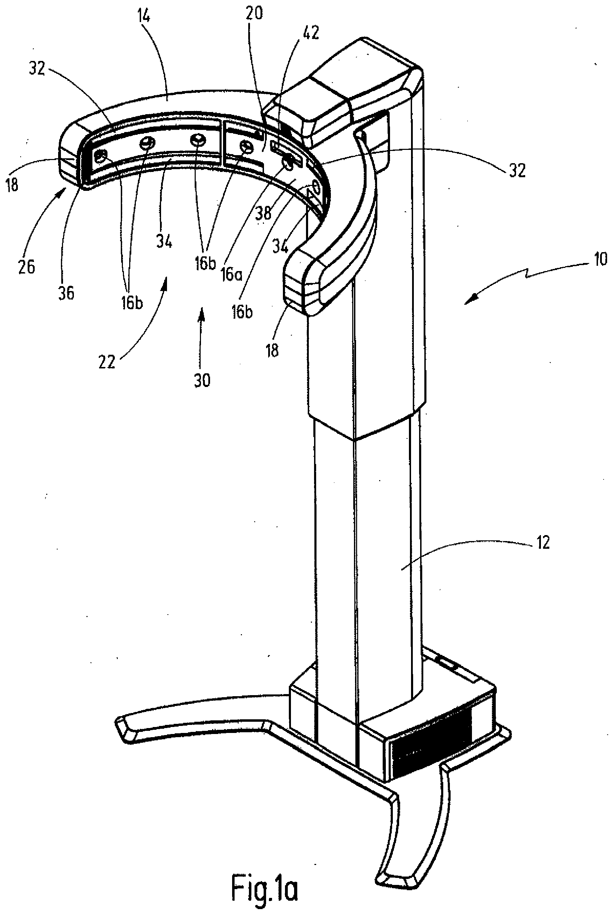

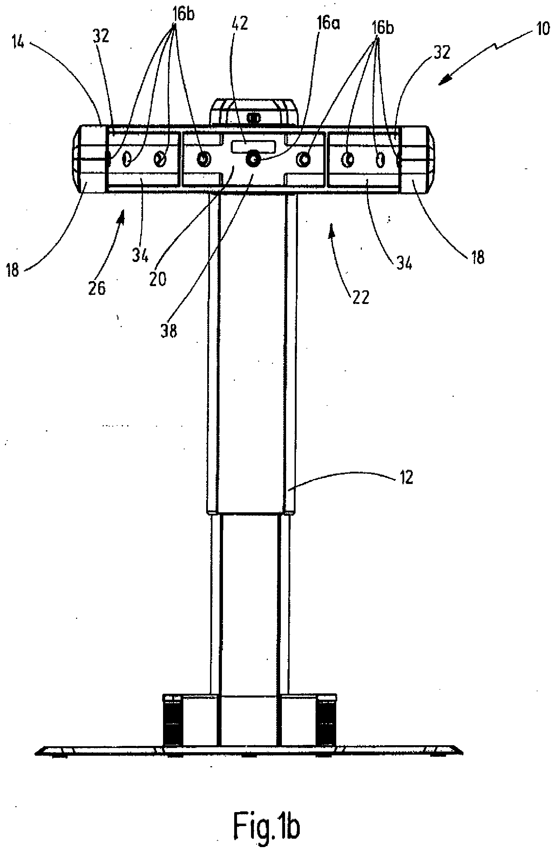

- Figur 1a, b: eine Vorrichtung zur Bestimmung von Zentrierparametern in perspektivischer Ansicht und in einer Ansicht von vorne.

Die in der Zeichnung dargestellte Vorrichtung 10 dient der Bestimmung von Zentrierparametern für die Brillenanpassung. Sie weist eine Säule 12 auf, die höhenverstellbar einen starren Kameraträger 14 trägt, welcher wiederum eine Anzahl Kameras 16a, 16b trägt. Der Kameraträger 14 ist in Draufsicht näherungsweise kreisförmig gebogen und erstreckt sich zwischen zwei freien Enden 18, welche im Abstand zueinander angeordnet sind. Nach vorne, also zur Säule 12 hin, und zu den Seiten umschließt eine Innenfläche 20 des Kameraträgers 14 einen Innenraum 22, in dem sich bei der Aufnahme von Bildern durch die Kameras 16a, 16b der Kopf eines Probanden befindet. Die Innenfläche 20 ist in einer Richtung, die zwischen den freien Enden 18 verläuft, konkav gebogen und weist beispielsweise die Form eines Abschnitts einer Zylindermantelfläche auf, wobei der Zylinder eine kreisrunde oder ovale Grundfläche haben kann. Um den Kameraträger 14 bezüglich des Kopfs des Probanden auf der richtigen Höhe positionieren zu können, ist in der Säule 12 eine nicht näher dargestellte Hubeinrichtung angeordnet, mit der der Kameraträger 14 motorisch angetrieben auf und ab bewegt werden kann.

Alle Kameras 16a, 16b sind äquiangular in einer sich zwischen den freien Enden 18 erstreckenden Kameraanordnung 26 angeordnet. Im vorliegenden Ausführungsbeispiel ist die Kameraanordnung 26 als Kamerareihe 26 ausgebildet, deren Kameras 16a, 16b sich alle in derselben Höhe befinden, wobei ihre optischen Achsen auf den Innenraum 22 gerichtet sind. Im vorliegenden Ausführungsbeispiel umfasst die Kamerareihe 26 eine in der Mitte des Kameraträgers 14 angeordnete Frontkamera 16a, deren optische Achse frontal auf das Gesicht des Probanden gerichtet ist, sowie acht paarweise symmetrisch bezüglich einer durch die optische Achse der Frontkamera 16a verlaufenden senkrechten Symmetrieebene angeordnete Seitenkameras 16b von denen jeweils vier von links und von rechts auf das Gesicht des Probanden gerichtet sind. Die Kameras 16a, 16b sind zudem kalibriert, so dass sie gleichzeitig kalibrierte Bilder des Probanden aufnehmen können. Die Kalibrierung umfasst die extrinsischen Eigenschaften wie die relative Ausrichtung ihrer optischen Achsen sowie die relative Anordnung zueinander im Raum, sowie ihre intrinsischen Eigenschaften, also die Eigenschaften der Kameras selbst, die definieren, wie ein Punkt im Raum, der sich im internen Koordinatensystem der jeweiligen Kamera befindet, auf die Koordinaten der Pixel des aufgenommenen Bildes abgebildet wird. Eine ausführliche Beschreibung der Kalibrierung von Kameras findet sich im Lehrbuch "Multiple View Geometry in Computer Vision" von Richard Hartley und Andrew Zisserman, 2. Auflage, Cambridge University Press 2004, und dort insbesondere auf Seite 8.

Der Kameraträger 14 umschließt den Innenraum 22 nur nach vorne, zur Säule 12 hin, und zu den Seiten, also links und rechts des Kopfes des Probanden. Nach oben, unten sowie zu einer Rückseite 30 hin ist er offen, wobei die freien Enden 18 zueinander einen Abstand von mindestens 25cm aufweisen, so dass sich der Proband bequem von der Rückseite aus nähern kann. Im gezeigten Ausführungsbeispiel beträgt der Abstand 70 bis 80cm, und die Kameras 16a, 16b sind in gleichen Winkelabständen zueinander angeordnet.

Zur Ausleuchtung des Innenraums 22 ist eine Beleuchtungseinrichtung mit einer oberhalb der Kamerareihe 26 verlaufenden oberen Lichtleiste 32 sowie einer unterhalb der Kamerareihe 26 verlaufenden unteren Lichtleiste 34 vorgesehen, welche jeweils eine Vielzahl von LEDs als Leuchtmittel aufweisen. Die obere Lichtleiste 32 und die untere Lichtleiste 34 erstrecken sich jeweils durchgehend oder mit Unterbrechungen über eine Länge, die mindestens so groß ist wie die Länge der in Umfangsrichtung zwischen den freien Enden 18 gemessenen Länge der Kamerareihe 26. Diese entspricht einem Umfangswinkel von mindestens 160 Grad. Nahe den freien Enden 18 sind die obere Lichtleiste 32 und die untere Lichtleiste 34 jeweils mittels einer vertikal verlaufenden weiteren Lichtleiste 36 miteinander verbunden. Die Kamerareihe 26 wird somit vollständig durch mindestens eine Reihe LEDs umrahmt. Die Vorrichtung 10 weist zudem eine in der Zeichnung nicht näher dargestellte Steuer- oder Regeleinrichtung auf, mit der die von den LEDs abgestrahlte Lichtintensität abhängig von der durch die Kameras 16a, 16b detektierten Lichtintensität gesteuert oder geregelt werden kann. Die LEDs der Lichtleisten 32, 34, 36 sind dabei zu Sektoren zusammengefasst, deren abgestrahlte Lichtintensitäten getrennt voneinander gesteuert bzw. geregelt werden können. Zudem ist vorgesehen, dass auch die von den einzelnen LEDs abgestrahlten Lichtintensitäten mittels der Steuer- oder Regeleinrichtung getrennt voneinander gesteuert oder geregelt werden können.

Um den Probanden richtig im Innenraum 22 positionieren zu können, sind die beiden der Frontkamera 16a nächstgelegenen Seitenkameras 16b dazu eingerichtet, den Abstand des Kopfs des Probanden von der Mitte 38 des Kameraträgers 14 zu messen. Mittels einer nicht näher dargestellten Anzeigeeinheit wird dem Probanden angezeigt, ob er richtig steht oder nicht. Die Anzeigeeinheit weist mehrere unterschiedlich eingefärbte Lichtquellen auf, die in einer Reihe angeordnet sind. Die mittlere Lichtquelle leuchtet grün, wenn der Proband richtig steht. Ausgehend von der mittleren Lichtquelle gibt es in jeder Richtung in dieser Reihenfolge eine gelbe, eine orangene und eine rote Lichtquelle, die entsprechend der Farbe anzeigt, wenn der Proband ein wenig, deutlich oder viel zu weit von der Mitte 38 des Kameraträgers 14 entfernt bzw. ein wenig, deutlich oder viel zu nah zur Mitte 38 steht. Um bei der Bestimmung der Zentrierparameter sicherzustellen, dass die Blickrichtung des Probanden ins Unendliche gerichtet ist, ist eine am Kameraträger 14 angeordnete Fixationseinrichtung 42 vorgesehen, die ein Fixationsmuster für den Probanden in Form eines Specklemusters erzeugt. Das Fixationsmuster ist etwas höher angeordnet als die Frontkamera 16a, so dass der Proband über diese hinwegblickt. Damit kann sein Gesicht im größtmöglichen Umfang aufgenommen werden.

Die Vorrichtung 10 eignet sich insbesondere auch zur Herstellung eines Avatars des Kopfs des Probanden, welcher zur Bestimmung der Zentrierparameter herangezogen werden kann. Zum diesem Zweck werden durch die Kameras 16a, 16b kalibrierte Bilder des Kopfs des Probanden ohne Brille bzw. Brillenfassung aufgenommen. Dabei ist es vorteilhaft, wenn die Bilder gleichzeitig aufgenommen werden. Mittels eines geeigneten Prozesses zur geometrischen Positionsbestimmung wie beispielsweise Triangulation, Stereo-Rekonstruktion, Multiview-Reconstruction oder Structure from Motion wird ein dreidimensionales Tiefenprofil des Kopfes erstellt, das diesen näherungsweise sehr gut abbildet. Der Kopf wird durch eine Vielzahl von Punkten abgebildet, die mittels eines Netzmusters miteinander verbunden werden können oder aber als Punktwolke gespeichert werden können. Bei der anschließenden Bestimmung der Zentrierparameter kann der so ermittelte Avatar herangezogen werden, um Zentrierparameter zu bestimmen, die aufgrund der geometrischen Eigenschaften der Brille bzw. Brillenfassung, die der Proband trägt, nicht oder nur näherungsweise bestimmt werden können. Beispielsweise kann ein breiter Fassungsbügel das Auge in einer Seitenaufnahme soweit verdecken, dass der Hornhautscheitelabstand nicht oder nur sehr ungenau bestimmt werden kann. Zudem können gefärbte oder stark spiegelnde Gläser die Augen nicht oder nur sehr schlecht erkennen lassen. Um dem zu begegnen, wird auf die von den Kameras 16a, 16b aufgenommenen Bilder des die Brille oder Brillenfassung tragenden Probanden das Tiefenprofil des Avatars projiziert und die Zentrierparameter, die aufgrund der durch die Brille bzw. Brillenfassung eingeschränkten Sicht nur ungenügend bestimmt werden können, werden mittels der Bilddaten des Avatars bestimmt. Dabei kann eine Anpassung des Avatars auf die Bilder des die Brille bzw. Brillenfassung tragenden Probanden zur Minimierung von Abweichungen erfolgen. Zudem kann der Avatar dazu herangezogen werden, virtuell Brillen bzw. Brillenfassungen anzuprobieren, indem die ihre Geometrie beschreibenden Daten auf den Avatar eingeblendet werden.

## Patentansprüche

1. Vorrichtung zur Bestimmung von Zentrierparametern für die Brillenanpassung mit einem Kameraträger (14), welcher einen nach oben, nach unten und zu einer Rückseite (30) hin offenen Innenraum (22) teilweise umschließt und mindestens drei Kameras (16a, 16b) trägt, die zwischen zwei freien Enden (18) des Kameraträgers (14) angeordnet und auf den Innenraum (22) gerichtet sind, wobei der Kameraträger (14) zur Beleuchtung des Innenraums (22) eine Beleuchtungseinrichtung (32, 34, 36) aufweist.

2. Vorrichtung nach Anspruch 1, **dadurch gekennzeichnet, dass** die Kameras in einer sich zwischen den freien Enden (18) des Kameraträgers (14) erstreckenden Kameraanordnung (26) angeordnet sind und dass sich die Beleuchtungseinrichtung (32, 34, 36) zwischen den freien Enden (18) über eine Länge erstreckt, die mindestens der Länge der Kameraanordnung (26) entspricht.

3. Vorrichtung nach Anspruch 2, **dadurch gekennzeichnet, dass** die Beleuchtungseinrichtung (32, 34, 36) mindestens eine Lichtleiste oder eine Leuchtmittelreihe mit einer Vielzahl von Leuchtmitteln aufweist.

4. Vorrichtung nach Anspruch 3, **dadurch gekennzeichnet, dass** die Beleuchtungseinrichtung eine erste, über der Kameraanordnung (26) angeordnete, und eine zweite, unter der Kameraanordnung (26) angeordnete Lichtleiste (32, 34) oder Leuchtmittelreihe aufweist.

5. Vorrichtung nach Anspruch 4, **dadurch gekennzeichnet, dass** die Beleuchtungseinrichtung die erste und die zweite Lichtleiste (32, 34) oder Leuchtmittelreihe jeweils nahe den freien Enden (18) verbindende, vertikal verlaufende weitere Lichtleisten (36) oder Leuchtmittelreihen aufweist.

6. Vorrichtung nach einem der vorangehenden Ansprüche, **dadurch gekennzeichnet, dass** sich die Anordnung der Kameras (16a, 16b) über einen Mittelpunktswinkel von mindestens 150 Grad erstreckt und/oder dass die optischen Achsen der den freien Enden (18) des Kameraträgers (14) am nächsten gelegenen Kameras (16b) einen Winkel von 150 Grad einschließen.

7. Vorrichtung nach einem der vorangehenden Ansprüche, **dadurch gekennzeichnet, dass** die freien Enden (18) des Kameraträgers (14) in einem Abstand von mindestens 25cm zueinander angeordnet sind.

8. Vorrichtung nach einem der vorangehenden Ansprüche, **gekennzeichnet durch** eine Steuer- oder Regeleinrichtung zum Steuern oder Regeln der Lichtintensität der Leuchtmittel in Abhängigkeit von der Helligkeit im Innenraum (22).

9. Vorrichtung nach Anspruch 8, **dadurch gekennzeichnet, dass** Sektoren der Beleuchtungseinrichtung (32, 34, 36) und/oder einzelne Leuchtmittel getrennt voneinander steuerbar oder regelbar sind.

10. Vorrichtung nach einem der vorangehenden Ansprüche, **gekennzeichnet durch** einen Abstandssensor zur Messung des Abstands des Kopfes eines Probanden zur Mitte des Kameraträgers (14) sowie eine Anzeigeeinheit zur Anzeige des Abstands.

11. Verfahren zur Brillenanpassung, wobei mindestens drei kalibrierte, aus unterschiedlichen Perspektiven aufgenommene erste Bilder des Kopfs eines Probanden ohne Brille bereitgestellt werden, wobei mittels eines Prozesses zur geometrischen Positionsbestimmung aus den ersten Bildern ein dreidimensionales Tiefenprofil zumindest eines Teils des Kopfs bestimmt und gespeichert wird.

12. Verfahren nach Anspruch 11, **dadurch gekennzeichnet, dass** mindestens ein zweites Bild des eine Brille oder eine Brillenfassung tragenden Kopfs aufgenommen wird, wobei das Tiefenprofil auf das mindestens eine zweite Bild projiziert wird und wobei Zentrierparameter aus Bilddaten des mindestens einen zweiten Bilds und aus Daten des auf das mindestens eine zweite Bild projizierten Tiefenprofils bestimmt werden.

13. Verfahren nach Anspruch 11 oder 12, **dadurch gekennzeichnet, dass** die Projektion des Tiefenprofils auf das mindestens eine zweite Bild mittels eines Optimierungsprozesses zur Minimierung von Abweichungen an das mindestens eine zweite Bild angepasst wird.

14. Computerprogramm mit Programmcode zur Durchführung aller Verfahrensschritte nach einem der Ansprüche 11 bis 13, wenn das Computerprogramm in einem Computer geladen und/oder in einem Computer ausgeführt wird.

15. Verwendung einer Vorrichtung (10) nach einem der Ansprüche 1 bis 11 zur Durchführung des Verfahrens nach einem der Ansprüche 11 bis 13.

16. Verfahren zur Bestimmung von Zentrierparametern für die Brillenanpassung, wobei ein Kopf eines eine Brille tragenden Probanden aus mindestens drei Aufnahmerichtungen aufgenommen wird und wobei der Kopf des Probanden derart beleuchtet wird, dass die Beleuchtungsstärke an jeder Stelle des Kopfes, die aufgenommen wird, um maximal +50% und -30% von einem vorgegebenen Sollwert abweicht.

17. Verfahren zur Bestimmung von Zentrierparametern für die Brillenanpassung nach Anspruch 16 unter Verwendung einer Vorrichtung nach einem der Ansprüche 1 bis 11, **dadurch gekennzeichnet, dass** die Beleuchtungseinrichtung (32, 34, 36) den Innenraum (20) so ausleuchtet, dass die Lichtintensität an jeder Stelle eines vorbestimmten Bereichs einer im Innenraum (22) angeordneten Zylindermantelfläche mit 20 cm Durchmesser um maximal +50% und -30% von einem vorgegebenen Basiswert abweicht.

18. Vorrichtung zur Bestimmung von Zentrierparametern für die Brillenanpassung mit mindestens drei zueinander kalibrierten Kameras (16a, 16b), welche derart zueinander angeordnet sind, dass sie jeweils ein Bild eines Kopfes eines eine Brille tragenden Probanden gleichzeitig aufnehmen können und mit einer Recheneinrichtung, welche eingerichtet ist, aus den Bildern mittels eines Prozesses zur geometrischen Positionsbestimmung ein dreidimensionales Tiefenprofil zumindest eines Teils des Kopfes zu bestimmen.
